# EUROPEAN PATENT SPECIFICATION

(11) **EP 1 270 565 B1**
(45) Date of publication and mention of the grant of the patent: **30.06.2004**
(21) Application number: 02020749.4
(22) Date of filing: 29.12.1997
(51) Int. Cl.: C07D 307/64

(54) **4-Substituted furan-2-sulfonamide and its use in the preparation of sulfonyl urea derivatives**
4-Substituiertes Furan-2-sulfonamid und dessen Verwendung in der Herstellung von Sulfonylharnstoff-Derivate
Furane-2-sulfonamide 4-substituée et son emploi dans la préparation de dérivés de sulfonylurée

(30) Priority: 29.01.1997 US 36979 P
(43) Date of publication of application: 02.01.2003
(62) Divisional of application: 97947201.6
(73) Proprietor: PFIZER INC., New York, N.Y. 10017 (US)
(72) Inventor: Dombroski, Mark Anthony, c/o Pfizer Global R&D, Groton, Connecticut 06340 (US); Eggler, James Frederick, c/o Pfizer Global R&D, Groton, Connecticut 06340 (US)
(74) Representative: Stuart, Peter Graham

(56) References cited:
- WO-A-93/08161

## Description

This invention relates to substituted sulfonamide derivatives useful as intermediates in the preparation of aryl and heteroaryl substituted sulfonyl ureas.

European Patent Application No. EP 0964849 disdoses compounds of formula I, which are useful in the treatment of meningitis and salpingitis, septic shock, disseminated intravascular coagulation, and/or adult respiratory distress syndrome, acute, or chronic inflammation, arthritis, cholangitis, colitis, encephalitis, endocarditis,; glomerulonephritis, hepatitis, myocarditis, pancreatitis, pericarditis, reperfusion injury, vasculitis, acute and delayed hypersensitivity, graft rejection, and graft-versus-host disease, auto-immune diseases including Type 1 diabetes mellitus and multiple sclerosis, periodonate diseases, interstitial pulmonary fibrosis, cirrhosis, systemic sclerosis, keloid formation, tumors which produce IL-1 as an autocrine growth factor, cachexia, Alzheimers disease, percussion injury, depression, atherosclerosis (including cardiomyopathy, myocarditis and heart failure) and osteoporosis in a mammal, including a human: 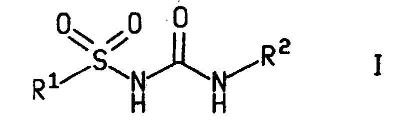 or a pharmaceutically acceptable salt thereof, wherein
R¹ is (C₁-C₆)alkyl optionally substituted by (C₁-C₆)alkylamino, (C₁-C₆)alkylthio, (C₁-C₆)alkoxy, trifluoromethyl, (C₆-C₁₀)aryl, (C₅-C₉)heteroaryl, (C₆-C₁₀)arylamino, (C₆-C₁₀)arylthio, (C₆-C₁₀)aryloxy, (C₅-C₉)heteroarylamino, (C₅-C₉)heteroarylthio, (C₅-C₉)heteroaryloxy, (C₆-C₁₀)aryl(C₆-C₁₀)aryl, (C₃-C₆)cycloalkyl, hydroxy(C₁-C₆)alkyl, (C₁-C₆)alkyl(hydroxymethylene), piperazinyl, (C₆-C₁₀)aryl(C₁-C₆)alkoxy, (C₅-C₉)heteroaryl(C₁-C₆)alkoxy, (C₁-C₆)acylamino, (C₁-C₆)acylthio, (C₁-C₆)acyloxy, (C₁-C₆)alkylsulfinyl, (C₆-C₁₀)arylsulfinyl, (C₁-C₆)alkylsulfonyl, (C₆-C₁₀)arylsulfonyl, amino, (C₁-C₆)alkylamino or ((C₁-C₆)alkyl)₂amino; or
R¹ and R² are each independently a group of the formula 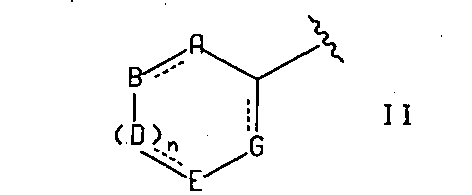 wherein the broken lines represent optional double bonds;
n is 0, 1, 2 or 3;
A, B, D, E and G are each independently oxygen, sulfur, nitrogen or CR⁵R⁶ wherein R⁵ and R⁶ are each independently selected from hydrogen, (C₁-C₆)alkyl optionally substituted by one or two groups selected from (C₁-C₆)alkylamino, (C₁-C₆)alkylthio, (C₁-C₆)alkoxy, hydroxy, cyano, perfluoro(C₁-C₆)alkyl, (C₆-C₁₀)aryl, (C₅-C₉)heteroaryl, (C₆-C₁₀)arylamino, (C₆-C₁₀)arylthio, (C₆-C₁₀)aryloxy wherein the aryl group is optionally substituted by (C₁-C₆)alkoxy, (C₁-C₆)acyl, carboxy, hydroxy or halo; (C₅-C₉)heteroarylamino, (C₅-C₉)heteroarylthio, (C₅-C₉)heteroaryloxy, (C₆-C₁₀)aryl(C₆-C₁₀)aryl, (C₃-C₆)cycloalkyl, hydroxy, piperazinyl, (C₆-C₁₀)aryl(C₁-C₆)alkoxy, (C₅-C₉)heteroaryl(C₁-C₆)alkoxy, (C₁-C₆)acylamino, (C₁-C₆)acylthio, (C₁-C₆)acyloxy, (C₁-C₆)alkylsulfinyl, (C₆-C₁₀)arylsulfinyl, (C₁-C₆)alkylsulfonyl, (C₆-C₁₀)arylsulfonyl, amino, (C₁-C₆)alkylamino or ((C₁-C₆)alkyl)₂amino; halo, cyano, amino, hydroxy, perfluoro(C₁-C₆)alkyl, perfluoro(C₁-C₆)alkoxy, (C₂-C₆)alkenyl, carboxy(C₂-C₆)alkenyl, (C₂-C₆)alkynyl, (C₁-C₆)alkylamino, ((C₁-C₆)alkyl)₂amino, (C₁-C₆)alkylsulfonylamido, (C₁-C₆)alkylsulfinyl, aminosulfonyl, (C₁-C₆)alkylaminosulfonyl, ((C₁-C₆)alkyl)₂aminosulfonyl, (C₁-C₆)alkylthio, (C₁-C₆)alkoxy, perfluoro(C₁-C₆)alkyl, (C₆-C₁₀)aryl, (C₅-C₉)heteroaryl, (C₆-C₁₀)arylamino, (C₆-C₁₀)arylthio, (C₆-C₁₀)aryl(C₁-C₆)alkoxy, (C₅-C₉)heteroarylamino, (C₅-C₉)heteroarylthio, (C₅-C₉)heteroaryloxy, (C₃-C₆)cycloalkyl, (C₁-C₆)alkyl(hydroxymethylene), piperidyl, pyridinyl; thienyl, furanyl, (C₁-C₆)alkylpiperidyl, (C₁-C₆)acylamino, (C₁-C₆)acylthio, (C₁-C₆)acyloxy, R⁷(C₁-C₆)alkyl wherein R⁷ is (C₁-C₆)acylpiperazino, (C₆-C₁₀)arylpiperazino, (C₅-C₉)heteroarylpiperazino, (C₁-C₆)alkylpiperazino, (C₆-C₁₀)aryl(C₁-C₆)alkylpiperazino, (C₅-C₉)heteroaryl(C₁-C₆)alkylpiperazino, morpholino, thiomorpholino, piperidino, pyrrolidino, piperidyl, (C₁-C₆)alkylpiperidyl, (C₆-C₁₀)arylpiperidyl, (C₅-C₉)heteroarylpiperidyl, (C₁-C₆)alkylpiperidyl(C₁-C₆)alkyl, (C₆-C₁₀)arylpiperidyl(C₁-C₆)alkyl, (C₅-C₉)heteroarylpiperidyl(C₁-C₆)alkyl or (C₁-C₆)acylpiperidyl;
or a group of the formula 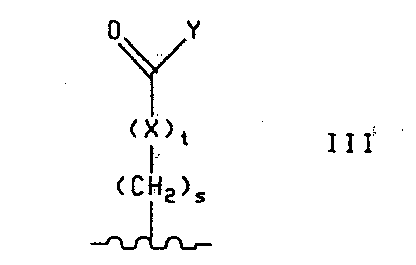 wherein s is 0 to 6;
t is 0 or 1;
X is oxygen or NR⁸ wherein R⁸ is hydrogen, (C₁-C₆)alkyl or (C₃-C₇)cycloalkyl(C₁-C₆)alkyl;
Y is hydrogen, hydroxy, (C₁-C₆)alkyl, optionally substituted by halo, hydroxy or cyano; (C₁-C₆)alkoxy, cyano, (C₂-C₆)alkynyl, (C₆-C₁₀)aryl wherein the aryl group is optionally substituted by halo, hydroxy, carboxy, (C₁-C₆)alkyl, (C₁-C₆)alkoxy; perfluoro(C₁-C₆)alkyl, (C₁-C₆)alkoxy(C₁-C₆)alkyl or NR⁹R¹⁰ wherein R⁹ and R¹⁰ are each independently selected from the group consisting of hydrogen, (C₁-C₆)alkyl optionally substituted by (C₁-C₆)alkylpiperidyl, (C₆-C₁₀)arylpiperidyl, (C₅-C₉)heteroarylpiperidyl, (C₆-C₁₀)aryl, (C₅-C₉)heteroaryl or (C₃-C₆)cycloalkyl; piperidyl, (C₁-C₆)alkylpiperidyl, (C₆-C₁₀)arylpiperidyl, (C₅-C₉)heteroarylpiperidyl, (C₁-C₆)acylpiperidyl, (C₆-C₁₀)aryl, (C₅-C₉)heteroaryl, (C₃-C₆)cycloalkyl, R¹¹(C₂-C₆)alkyl, (C₁-C₅)alkyl(CHR¹¹)(C₁-C₆)alkyl wherein R¹¹ is hydroxy; (C₁-C₆)acyloxy, (C₁-C₆)alkoxy, piperazino, (C₁-C₆)acylamino, (C₁-C₆)alkylthio, (C₆-C₁₀)arylthio, (C₁-C₆)alkylsulfinyl, (C₆-C₁₀)arylsulfinyl, (C₁-C₆)alkylsulfoxyl, (C₆-C₁₀)arylsulfoxyl, amino, (C₁-C₆)alkylamino, ((C₁-C₆)alkyl)₂amino, (C₁-C₆)acylpiperazino, (C₁-C₆)alkylpiperazino, (C₆-C₁₀)aryl(C₁-C₆)alkylpiperazino, (C₅-C₉)heteroaryl(C₁-C₆)alkylpiperazino, morpholino, thiomorpholino, piperidino or pyrrolidino; R¹²(C₁-C₆)alkyl, (C₁-C₅)alkyl(CHR¹²)(C₁-C₆)alkyl wherein R¹² is piperidyl or (C₁-C₆)alkylpiperidyl; and CH(R¹³)COR¹⁴ wherein R¹⁴ is as defined below and R¹³ is hydrogen, (C₁-C₆)alkyl, (C₆-C₁₀)aryl(C₁-C₆)alkyl, (C₅-C₉)heteroaryl(C₁-C₆)alkyl, (C₁-C₆)alkylthio(C₁-C₆)alkyl, (C₆-C₁₀)arylthio(C₁-C₆)alkyl, (C₁-C₆)alkylsulfinyl(C₁-C₆)alkyl, (C₆-C₁₀)arylsulfinyl(C₁-C₆)alkyl, (C₁-C₆)alkylsulfonyl(C₁-C₆)alkyl, (C₆-C₁₀)arylsulfonyl(C₁-C₆)alkyl, hydroxy(C₁-C₆)alkyl, amino(C₁-C₆)alkyl, (C₁-C₆)alkylamino(C₁-C₆)alkyl, ((C₁-C₆)alkylamino)₂(C₁-C₆)alkyl, R¹⁵R¹⁶NCO(C₁-C₆)alkyl or R¹⁵OCO(C₁-C₆)alkyl wherein R¹⁵ and R¹⁶ are each independently selected from the group consisting of hydrogen, (C₁-C₆)alkyl, (C₆-C₁₀)aryl(C₁-C₆)alkyl and (C₅-C₉)heteroaryl(C₁-C₆)alkyl; and R¹⁴ is R¹⁷O or R¹⁷R¹⁸N wherein R¹⁷ and R¹⁸ are each independently selected from the group consisting of hydrogen, (C₁-C₆)alkyl, (C₆-C₁₀)aryl(C₁-C₆)alkyl and (C₅-C₉)heteroaryl(C₁-C₆)alkyl;
or a group of the formula 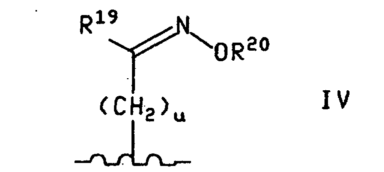 wherein u is 0, 1 or 2;
R¹⁹ is hydrogen, (C₁-C₆)alkyl or perfluoro(C₁-C₆)alkyl;
R²⁰ is hydrogen, (C₁-C₆)alkyl, (C₁-C₆)carboxyalkyl or (C₆-C₁₀)aryl(C₁-C₆)alkyl;
or a group of the formula 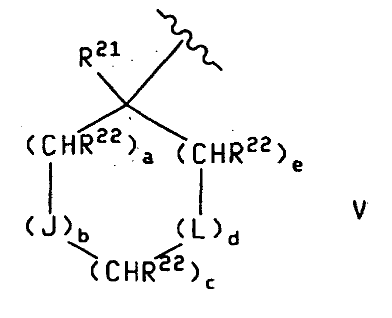 wherein a is 0, 1 or 2;
b is 0 or 1;
c is 1, 2 or 3;
d is 0 or 1;
e is 0, 1 or 2;
J and L are each independently oxygen or sulfur;
R²¹ is hydrogen, hydroxy, fluoro, (C₁-C₆)alkyl, (C₁-C₆)alkoxy, halo(C₁-C₆)alkyl, amino, (C₁-C₆)acylamino or NR²⁶R²⁷ wherein R²⁶ and R²⁷ are each independently selected from hydrogen, (C₁-C₆)alkyl or (C₆-C₁₀)aryl; and
R²² is hydrogen, (C₁-C₆)alkyl optionally substituted by hydroxy, halo, (C₁-C₆)alkylthio, (C₁-C₆)alkylsulfinyl or (C₁-C₆)alkylsulfonyl;
or when n is 1 and B and D are both CR⁵, the two R⁵ groups may be taken together with the carbons to which they are attached to form a group of the formula 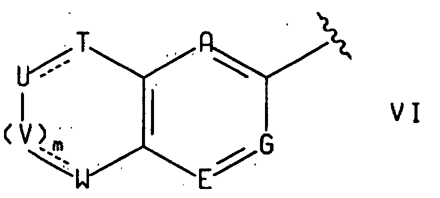 wherein the broken lines represent optional double bonds;
m is 0 or 1; and
T, U, V and W are each independently oxygen, sulfur, CO, nitrogen or CR⁵R⁶ wherein R⁵ and R⁶ are as defined above;
or when A and B, or when n is 1 and B and D, or D and E, or E and G, are both CR⁵, the two R⁵ groups may be taken together with the adjacent carbons to which they are attached to form a (C₅-C₆)cycloalkyl group optionally substituted by hydroxy or a benzo group;
or when n is 1 and D and E are both CR⁵, the two R⁵ groups may be taken together with the adjacent carbons to which they are attached to form a group of the formula 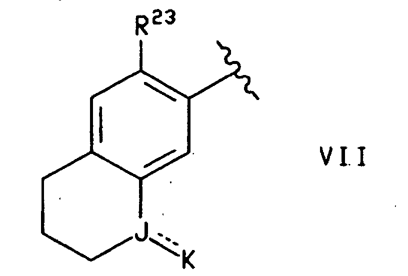 wherein the broken line represents an optional double bond;
R²³ is hydrogen, (C₁-C₆)alkyl, halo, amino or (C₁-C₆)alkoxy;
J is C or SO;
K is oxygen, NR²⁴ wherein R²⁴ is hydroxy, (C₁-C₆)alkoxy or (C₆-C₁₀)aryl(C₁-C₆)alkoxy; or hydroxy;
or R²⁵SO₂ wherein R²⁵ is defined as R¹ above or (C₃-C₇)cycloalkylamino; and
with the proviso that the groups of formulas II and VI cannot have two oxygens, two sulfurs or an oxygen and sulfur defined in adjacent positions;
with the proviso that R² must be aromatic;
with the proviso that when either a or e is 0, the other must be 1;
with the proviso that when b and d are 1, the sum of a, c and e cannot be 6 or 7; and
with the proviso that when A, B, D, E, G, T, U, V and W represent an sp² carbon, R⁶ does not exist.

Specific preferred compounds of formula **I** include the following:
1-(1,2,3,5,6,7-Hexahydro-s-indacen-4-yl)-3-[4-(1-hydroxy-1-methyl-ethyl)-furan-2-sulfonyl]-urea;
1-(1,2,3,5,6,7-Hexahydro-4-aza-s-indacen-8-yl)-3-[4-(1-hydroxy-1-methyl-ethyl)-furan-2-sulfonyl]-urea;
1-(2,6-Diisopropyl-phenyl)-3-[4-(1-hydroxy-1-methyl-ethyl)-furan-2-sulfonyl]-urea;
1-(8-Chloro-1,2,3,5,6,7-hexahydro-s-indacen-4-yl)-3-[4-(1-hydroxy-1-methyl-ethyl)-furan-2-sulfonyl]-urea;
1-(8-Fluoro-1,2,3,5,6,7-hexahydro-s-indacen-4-yl)-3-[4-(1-hydroxy-1-methyl-ethyl)-furan-2-sulfonyl]-urea;

### Summary of the Invention

The present invention relates to a compound, 4-(1-hydroxy-1-methyl-ethyl)furan-2-sulfonamide.

The present invention further relates to a process for the preparation of a compound selected from:
1-(4-chloro-2,6-diisopropyl-phenyl)-3-[4-(1-hydroxy-1-methyl-ethyl)-furan-2-sulfonyl]-urea;
1-(8-chloro-1,2,3,5,6,7-hexahydro-s-indacen-4-yl)-3-[4-(1-hydroxy-1-methyl-ethyl)-furan-2-sulfonyl]-urea;
1-(1,2,3,5,6,7-hexahydro-s-indacen-4-yl)-3-[4-(1-hydroxy-1-methyl-ethyl)-furan-2-sulfonyl]-urea; and
1-(2,6-diisopropyl-phenyl)-3-[4-(1-hydroxy-1-methyl-ethyl)-furan-2-sulfonyl]-urea, comprising the reaction of the compound 4-(1-hydroxy-1-methyl-ethyl)furan-2-sulfonamide with the corresponding isocyanate.

### Detailed Description of the Invention

The sulfonamide of the invention may be converted to the corresponding sulfonyl urea compound by reaction with an isocyanate compound in the presence of a base, such as sodium hydride, sodium hydroxide, triethylamine or 1,8-diazabicyclo[5.4.0]undec-7-ene, and a polar solvent, such as tetrahydrofuran, acetone or dimethylformamide. The reaction mixture so formed is heated to reflux for a time period between about 10 hours to about 14 hours, preferably about 12 hours.

The present invention is illustrated by the following preparations and examples, but is not limited to the details thereof.

### PREPARATION A

### 4-Chloro-2,6-diisopropyaniline

To a stirred solution of 2,6-diisopropylaniline (47 grams) in N,N-dimethylformamide (886 mL) was added N-chlorosuccinimide (37.3 grams) and the mixture was stirred overnight. The resulting dark red solution was poured into water (12 L) and extracted with diethyl ether. The combined ether extracts were washed with water and brine, dried over:anhydrous sodium sulfate and concentrated in vacuo. The resulting dark red oil was purified by filtration through silica gel, eluting with 6:1 hexane/methylene chloride to afford 32 grams of the title compound. ¹H NMR δ 7.02 (s, 2), 3.71 (br s, 2), 2.91 (qq, 2, J = 6.9 Hz), 1.27 (d, 6, J = 6.9 Hz), 1.27 (d, 6, J = 6.9 Hz).

### PREPARATION B

### 4-Chloro-2,6-diisopropylphenylisocyanate

To a stirred solution of 4-chloro-2,6-diisopropylaniline (32 grams) and triethylamine (7.8 mL) in tetrahydrofuran (505 mL) was added triphosgene (14.9 grams). The mixture was refluxed for two hours with stirring. The tetrahydrofuran was then revolved in vacuo and the resulting oil was taken up in pentane and filtered through silica gel to afford 33.3 grams of the, product. ¹H NMR δ 7.18 (s, 2), 3.22 (qq, 2, J = 7.1 Hz), 1.25 (d, 6, J = 7.1 Hz), 1.25 (d, 6, J = 7.1 Hz).

### PREPARATION C

### 3,5,6,7-Tetrahydro-2H-s-indacen-1-one

Concentrated sulfuric acid (550 mL) was added dropwise, with stirring over a time period of 2 hours to 137 grams of 3-chloro-1-indan-5-yl-propan-1-one. The resulting thick black solution was heated to 90°C until hydrogen chloride evolution ceased (usually 1-4 hours). The mixture was then cooled to room temperature and poured onto 5 kg of ice. The resulting slurry was stirred overnight and filtered. The solid was washed with water until the water ran clear through the filter. The tan solid was then dried in vacuo and recrystallized from hexane to afford 90 grams of the title compound, m.p. 72.4-74.8°C.

### PREPARATION D

### 1,2,3,5,6,7-Hexahydro-s-indacene

A mixture of 3,5,6,7-tetrahydro-2H-s-indacen-1-one (90 grams), ethanol (1L), 10% palladium on carbon (1-2 grams) and concentrated hydrochloric acid (50 mL) was hydrogenated on a Parr shaker at room temperature until hydrogen uptake ceased. The mixture was filtered through a Celite pad. The pad was washed with 1L diethyl ether. The filtrate was diluted. with water and the organic phase was separated. The aqueous phase was extracted with 1L of ether, and the combined ether extracts were washed with water, saturated sodium bicarbonate solution and brine. The ether extracts were dried over anhydrous sodium sulfate, and concentrated in vacuo. The resulting pale yellow solid was recrystallized from methanol to afford 61 grams of the title compound as colorless crystals, m.p. 56.6-58.5°C.

### PREPARATION E

### 1-(1,2,3,5,6,7-Hexahydro-s-indacen-4-yl)-ethanone

To a stirred solution of 1,2,3,5,6,7-hexahydro-s-indacene (30 grams) and acetyl chloride (14.2 mL) in 120 mL of benzene at 0°C was added 30 grams of aluminum chloride over a period of 1 hour. The cooling bath was removed and the solution was warmed to room temperature and stirred for 4 hours. The deep red mixture was then poured onto a mixture of 270 grams of ice and 50 mL of concentrated hydrochloric acid. The layers were separated and the aqueous phase was extracted with diethyl ether. The combined organic phases were washed with saturated sodium bicarbonate solution and brine, dried over anhydrous sodium sulfate and concentrated in vacuo to afford an orange solid which was recrystallized from hexane to give 34 grams of the title compound, m.p. 69.1-76.1°C.

### PREPARATION F

### 1-(1,2,3,5,6,7-Hexahydro-s-indacen-4-yl)-ethanone oxide

A mixture of 1-(1,2,3,5,6,7-hexahydro-s-indacen-4-yl)-ethanone (33 grams), ethanol (250 mL) hydroxylamine hydrochloride (58.5 grams) and pyridine (80 mL) was heated to reflux for a period of 12 hours. The mixture was then cooled to room temperature and concentrated in vacuo. The residue was then treated with 500 mL of water and extracted with chloroform-methanol. The organic phase was dried over anhydrous sodium sulfate and concentrated in vacuo to afford 32 grams of the title compound as a mixture of E and Z isomers, 178.6-182.3°C.

### PREPARATION G

### N-(1,2,3,5,6,7-Hexahydro-s-indacen-4-yl)-acetamide

A mixture of 1-(1,2,3,5,6,7-hexahydro-s-indacen-4-yl)-ethanone oxime (85 grams) in 270 mL of trifluoroacetic acid was added dropwise to a stirred refluxing solution of 90 mL of trifluoroacetic acid over a period of 1/2 hour. The resulting purple solution was then. refluxed for 1 hour. The solution was cooled to room temperature and the trifluoroacetic acid was removed in vacuo. The dark solid was triturated with ethyl acetate/hexanes to afford 83 grams of a grey solid which was used without further purification, m.p. 257.4-259.1°C.

### PREPARATION H

### 1,2,3,5,6,7-Hexahydro-s-indacen-4-ylamine

A slurry of N-(1,2,3,5,6,7-hexahydro-s-indacen-4-yl)-acetamide (110 grams) in YY mL of 25% sulfuric acid was treated with enough ethanol to make a solution (ca YY mL). The resulting solution was heated to reflux for a period of 2 days. The resulting black solution was treated with charcoal at reflux, filtered hot and cooled to 0°C. The solution was then cautiously neutralized with 20% sodium hydroxide solution. The resulting slurry was then filtered and washed with water until the filtrate ran neutral. The tan solid was then isolated and dried in vacuo to give 80 grams of the title compound, m.p. 94.5-96.6°C, which was used without further purification. If necessary, the title compound can be recrystallized from methanol to afford a white solid.

### PREPARATION I

### 4-Isocyanato-1,2,3,5,6,7-Hexahydro-s-indacene

To a stirred solution of 1,2,3,5,6,7-hexahydro-s-indacen-4-ylamine (77 grams) in tetrahydrofuran (1.5 L) and triethylamine (68.3 mL) was added triphosgene (43.9 grams) in one portion. The mixture was heated to reflux for 1/2 hour. then cooled to room temperature. The tetrahydrofuran was removed under reduced pressure, and the residue was taken up in pentane and filtered through a plug of silica get. Removal of the pentane in vacuo afforded 80 grams of a white solid, m.p. 35.0-36.2°C.

Preparations J and K illustrate general methods of preparing the compound of the invention.

### PREPARATION J

### 3-(1-Hydroxy-1-methyl)ethylfuran

To a stirred solution of 24.97 MI of methyl magnesium bromide (3M solution in diethyl ether) at 0°C was added 4.82 MI of ethyl 3-furoate in diethyl ether. The mixture was heated. gently using a warm water bath for 30 minutes. Upon completion, the mixture was poured into ice water, carefully acidified using a buffered solution, and extracted with diethyl ether. The ether extracts were combined, washed with brine, dried over sodium sulfate, and concentrated in vacuo. The tertiary alcohol furan was purified using flash column chromatography with 6:1 hexane/ethyl acetate. Recovery: 2.89 grams (64%). ¹H NMR (400 MHz, Acetone-d₆) δ 1.45 (s, 3H), 1.46 (s, 3H), 3.89 (br s, 1H), 6.45 (br s, 1H), 7.41 (br s, 1H), 7.42 (br s, 1H).

### PREPARATION K

### 2-Aminosulfonyl-3-(1-hydroxy-1-methyl)ethylfuran

To a stirred mixture of 2.89 grams of tertiary alcohol furan in THF at -78°C was added 17.19 MI of methyl lithium (1.4M solution in diethyl ether) followed 5 minutes later by 18.51 MI of sec-butyl lithium (1.3M solution in cyclohexanes). The mixture continued to stir at -78°C for 40 minutes and 5.02 MI of liquid SO₂ was added. The temperature was maintained at -78°C for 5 minutes and was then warmed to room temperature with continued stirring for 2 hours. The THF was then removed in vacuo and the lithium sulfinate was dissolved in 76.4 MI of water followed by addition of 7.78 grams of hydroxylamine o-sulfonic acid and 31 grams of sodium acetate. This mixture stirred at room temperature overnight and was extracted with ethyl acetate. The ethyl acetate extracts were combined, washed with brine, dried over sodium sulfate, and concentrated in vacuo. The sulfonamide was purified using flash column chromatography with 2:1 hexane/ethyl acetate. Recovery: 1.91 grams (41%) m.p. 110.1-111.6°C.

### EXAMPLE 1

### 4-(Hydroxy-1-methyl-ethyl)furan-2-sulfonamide

The title compound can be prepared by a method analogous to that described in preparation K.

The compound of the invention can be reacted with isocyanate derivatives to prepare the sulfonyl urea compounds of examples 3-6. Example 2 illustrates a general method for the preparation of sulfonyl urea compounds.

### EXAMPLE 2

### 1-(4-Chloro-2,6-diisopropyl-phenyl)-3-[3-(1-hydroxy-1-methylethyl)-benzenesulfonyl]-urea

To a stirred solution of 2-(3-aminosulfonylphenyl)-propan-2-ol (26.5 grams) in tetrahydrofuran was added sodium hydride (5.2 grams of a 60% dispersion in mineral oil) in several portions. Once hydrogen evolution had subsided, 4-chloro-2,6-dissopropylphenylisocyanate (30.8 grams) was added in one portion, and the resulting mixture was heated to reflux for twelve hours. The mixture was then cooled to room temperature and concentrated in vacuo. The resulting foam was dissolved. in water, made basic with 1N sodium hydroxide and extracted with two portions of a 1:1 ration of ether/hexane. The aqueous layer. was acidified with 1N hydrochloric acid, and the resulting white solid was filtered, washed with water and dried. This afforded 50 grams of a white solid which was recrystallized from wet ethyl acetate/hexane afforded the title compound, melting point 160.5-162.0°C.

The titled compounds of Example 3-6 were prepared by a method analogous to that described in Example 2 using the reagents indicated.

### EXAMPLE 3

### 1-(2,6-Diisopropyl-phenyl)-3-[4-(1-hydroxy-1-methyl-ethyl)-furan-2-sulfonyl]-urea

4-(1-Hydroxy-1-methyl-ethyl)-furan-2-sulfonamide; 2,6-Diisopropyl-phenyl isocyanate. mp: 121.3-126.4°C.

### EXAMPLE 4

### 1-(1,2,3,5,6,7-Hexahydro-s-indacen-4-yl)-3-[4-(1-hydroxy-1-methyl-ethyl)-furan-2-sulfonyl]-urea

4-(1-Hydroxy-1-methyl-ethyl)-furan-2-sulfonamide; 4-Isocyanato-1,2,3,5,6,7-hexahydro-s-indacene. mp: 153.8-154.4°C.

### EXAMPLE 5

### 1-(8-Chloro-1,2,3,5,6,7-hexahydro-s-indacen-4-yl)-3-[4-(1-hydroxy-1-methyl-ethyl)-furan-2-sulfonyl]-urea

4-(1-Hydroxy-1-methyl-ethyl)-furan-2-sulfonamide; 4-Chloro-8-isocyanato-1,2,3,5,6,7-hexahydro-s-indacene. mp: 163.7°C (decomposed).

### EXAMPLE 6

### 1-(4-Chloro-2,6-diisopropyl-phenyl)-3-[4-(1-hydroxy-1-methyl-ethyl)-furan-2-sulfonyl]-urea

4-(1-Hydroxy-1-methyl-ethyl)-furan-2-sulfonamide; 4-Chloro-2,6-diisopropyl-phenyl isocyanate. mp: 127.4-129.2°C.

## Claims

1. The compound 4-(1-hydroxy-1-methyl-ethyl)-furan-2-sulfonamide and salts thereof.

2. A process for the preparation of a compound selected from:
1-(4-chloro-2,6-diisopropyl-phenyl)-3-[4-(1-hydroxy-1-methyl-ethyl)-furan-2-sulfonyl]-urea;
1-(8-chloro-1,2,3,5,6,7-hexahydro-s-indacen-4-yl)-3-[4-(1-hydroxy-1-methyl-ethyl)-furan-2-sulfonyl]-urea;
1-(1,2,3,5,6,7-hexahydro-s-indacen-4-yl)-3-[4-(1-hydroxy-1-methyl-ethyl)-furan-2-sulfonyl]-urea; and
1-(2,6-diisopropyl-phenyl)-3-[4-(1-hydroxy-1-methyl-ethyl)-furan-2-sulfonyl]-urea, comprising the reaction of the compound of claim 1 with the correspondong isocyanate.

## Patentansprüche

1. Die Verbindung 4-(1-Hydroxy-1-methyl-ethyl)-furan-2-sulfonamid und Salze derselben.

2. Verfahren zur Herstellung einer Verbindung, die ausgewählt ist aus:
1-(4-Chlor-2,6-diisopropyl-phenyl)-3-[4-(1-hydroxy-1-methyl-ethyl)-furan-2-sulfonyl]-harnstoff;
1-(8-Chlor-1,2,3,5,6,7-hexahydro-s-indacen-4-yl)-3-[4-(1-hydroxy-1-methyl-ethyl)-furan-2-sulfonyl]-harnstoff;
1-(1,2,3,5,6,7-Hexahydro-s-indacen-4-yl)-3-[4-(1-hydroxy-1-methyl-ethyl)-furan-2-sulfonyl]-harnstoff; und
1-(2,6-Diisopropyl-phenyl)-3-[4-(1-hydroxy-1-methylethyl)-furan-2-sulfonyl]-harnstoff,
wobei das Verfahren die Reaktion der Verbindung nach Anspruch 1 mit dem entsprechenden Isocyanat umfasst.

## Revendications

1. Composé consistant en 4-(1-hydroxy-1-méthyléthyl)-furanne-2-sulfonamide et ses sels.

2. Procédé pour la préparation d'un composé choisi entre les suivants :
1-(4-chloro-2,6-diisopropylphényl)-3-[4-(1-hydroxy-1-méthyléthyl)-furanne-2-sulfonyl]-urée ;
1-(8-chloro-1,2,3,5,6,7-hexahydro-s-indacène-4-yl)-3-[4-(1-hydroxy-1-méthyléthyl)-furanne-2-sulfonyl]-urée ;
1-(1,2,3,5,6,7-hexahydro-s-indacène-4-yl)-3-[4-(1-hydroxy-1-méthyléthyl)-furanne-2-sulfonyl]-urée ; et
1-(2,6-diisopropylphényl)-3-[4-(1-hydroxy-1-méthyléthyl)-furanne-2-sulfonyl]urée, comprenant la réaction d'un composé suivant la revendication 1 avec l'isocyanate correspondant.
